# EUROPÄISCHE PATENTSCHRIFT

(11) **EP 0 973 430 B1**
(45) Veröffentlichungstag und Bekanntmachung des Hinweises auf die Patenterteilung: **14.07.2004**
(21) Anmeldenummer: 98921403.6
(22) Anmeldetag: 27.03.1998
(51) Int. Cl.: A61B 1/00, A61B 1/04

(54) **ENDOSKOP MIT LÄNGENAUSGLEICH BEI THERMISCHER BELASTUNG**
ENDOSCOPE WITH LENGTH CORRECTION DURING THERMAL STRESS
ENDOSCOPE AVEC COMPENSATION DE LONGUEUR EN CAS DE CHARGE THERMIQUE

(30) Priorität: 29.03.1997 DE 19713275
(43) Veröffentlichungstag der Anmeldung: 26.01.2000
(73) Patentinhaber: Karl Storz GmbH & Co. KG, 78532 Tuttlingen (DE)
(72) Erfinder: RUDISCHHAUSER, Jürgen, D-78532 Tuttlingen (DE); RENNER, Klaus, D-78576 Liptingen (DE); HÖFIG, Siegfried, D-78532 Tuttlingen (DE)
(74) Vertreter: Weller, Wolfgang, Dr.
(86) Internationale Anmeldenummer: PCT/EP1998/001826
(87) Internationale Veröffentlichungsnummer: WO 1998/043529

(56) Entgegenhaltungen:
- WO-A-96/05764
- DE-A- 3 708 124

## Beschreibung

Die Erfindung betrifft ein Endoskop mit einem Außenrohr, das mit einem Optikkopf verbunden ist, welcher eine Okularmuschel trägt oder eine Adaptereinrichtung für ein Kamerasystem oder eine integrierte Miniaturkamera, ferner mit einem im Außenrohr angeordneten, sich in den Optikkopf hinein erstreckenden und dort abgestützten Innenrohr, das optische Bauelemente trägt, wobei Außenrohr und Innenrohr am distalen Ende des Endoskops starr und dichtend miteinander verbunden sind.

Derartige Endoskope sind allgemein bekannt und werden in dieser Bauart beispielsweise von der Anmelderin vertrieben.

Im Innenrohr sind optische Bauelemente, beispielsweise Stablinsen, angeordnet, weitere Bauelemente erstrecken sich bis in den Optikkopf hinein. Vom proximalen Ende der Okularmuschel her, die ein Fenster trägt, kann durch die Optik beobachtet werden. Bei anderen Ausführungen ist statt der Okularmuschel eine Adaptereinrichtung für eine Kamera vorgesehen oder eine Miniaturkamera ist direkt integriert. Diese drei Ausführungen stellen die Beobachtungselemente des Optikkopfes dar. Das Außenrohr, das das Innenrohr umgibt, umgrenzt einen Ringraum um die Außenseite des Innenrohres, der dazu dient, um Lichtleiter, beispielsweise Glasfasern zum distalen Ende des Endoskops zu führen, um die zu beoachtende Stelle zu beleuchten. Die Glasfasern werden üblicherweise über einen radial abstehenden Stutzen am Optikkopf in diesen Ringraum zugeführt. Am distalen Ende sind Außenrohr und Innenrohr über eine dichte Verbindung fest miteinander verbunden, so daß von dieser Seite keine Flüssigkeiten oder Gase in den Innenraum des Endoskops eintreten können.

Nach der Durchführung von Operationen müssen die Endoskope sterilisiert werden, wozu sie in Autoklaven auf Temperaturen im Bereich von 130 - 140°C erhitzt werden.

Nachdem minimal invasive Eingriffe zu Routineeingriffen geworden sind, und beispielsweise in Krankenhäusern täglich zahlreiche endoskopisch überwachte Operationen durchgeführt werden, sind die Endoskope häufig im Einsatz und sind demzufolge starken mechanischen Beanspruchungen, insbesondere beim Autoklavieren unterworfen. Um Endoskope nach einer Operation möglichst rasch wieder zur Verfügung zu haben, haben sich sogenannte Blitz-Autoklaviertechniken entwickelt, bei denen die gesamten Endoskope auf 143°C erwärmt und anschließend mit kaltem Wasser abgeschreckt werden. Diese extremen Temperaturwechsel müssen von der mechanischen Seite her aufgefangen werden, damit durch Wärmedehnungen kein Schaden an der Optik entsteht, beispielsweise daß diese undicht wird und Feuchtigkeit in das optische System eintreten kann. Daher muß für solche Temperaturschocks ein Ausdehnungsausgleich geschaffen werden. Dieser Ausgleich ist im wesentlichen ein Längendehnungsausgleich der langerstreckten Endoskope.

Bei einer bekannten Lösung ist das proximale Ende des Außenrohrs axial beweglich im Optikkopf gelagert, ein entsprechender O-Ring sorgt für einen dichtenden Abschluß. Dadurch ist ein Längenausdehnungsausgleich bei den zuvor erwähnten Temperaturschocks geschaffen.

Nachteilig an dieser Konstruktion ist, daß aufgrund der beweglichen Lagerung die mechanische Stabilität der Verbindung zwischen Außenrohr und Optikkopf nicht auf Dauer gewährleistet ist. Beim Ablegen eines Endoskops wirkt auf die Verbindungsstelle ein Drehmoment ein, da der Optikkopf üblicherweise einen größeren Durchmesser als das Außenrohr aufweist und über eine Stufe in den schlanken Endoskopschaft übergeht. Lockert sich diese Verbindung zwischen Außenrohr und Optikkopf, ist nicht nur die mechanische Stabilität beeinträchtigt, sondern es besteht auch die Möglichkeit, daß in den Innenraum des Optikkopfs Feuchtigkeit eindringen kann und das optische System beeinträchtigt.

Aus der WO-A-9 605 764 ist ein Endoskop bekannt, bei dem ein Bündel der optischen Fasern im Optikkopf von einem Gleitstück aufgenommen sind, das axial beweglich längs der Innenseite des Gehäuses des Kopfes gleiten kann. Dadurch ist eine thermische Längenausdehnungsmöglichkeit gegeben. Von der Außenseite her können jedoch Kontaminationen in den Innenraum eindringen.

Aus der DE-A-3 708 124 ist ein Endoskop mit zwei jeweils luftdichten Schalen, nämlich dem Endoskopkörper und dem optischen Beobachtungssystem beschrieben. Durch eine doppelte Abdichtung oder gegebenenfalls mit Hilfe eines zusätzlichen Trockenmittels soll ein Beschlagen des optischen Beobachtungssystems verhindert werden.

Bei einer weiteren bekannten Bauweise, wie sie von der Firma Richard Wolf GmbH bekanntgeworden ist, ist das proximale Ende des Innenrohres über einen O-Ring dichtend, jedoch axial beweglich, an der Innenseite des Optikkopfes abgestützt. Wird diese Dichtungsstelle aufgrund zahlreicher Längenausdehnungen bei den Autoklavierzyklen undicht, besteht die Gefahr, daß direkt Feuchtigkeit in das Innenrohr und somit in die Optik eindringen kann.

Es ist daher Aufgabe der vorliegenden Erfindung, hier Abhilfe zu schaffen und ein Endoskop der eingangs genannten Art dahingehend weiterzubilden, daß auf Dauer, insbesondere auch nach zahlreichen Blitz-Autoklavierzyklen, ein mechanisch stabiles Endoskop verbleibt und daß, bei vorhandenen Längenausdehnungsmöglichkeit, ausgeschlossen ist, daß Kontaminationen in die optischen Bauelemente eindringen können.

Erfindungsgemäß wird die Aufgabe dadurch gelöst, daß Außenrohr, Optikkopf und Okularmuschel oder die Adaptereinrichtung für eine Kamera oder die integrierte Miniaturkamera zu einer festen und zwischen diesen Teilen dichtenden, nach außen jedoch nicht abgeschlossenen ersten Baueinheit zusammengefügt sind, und daß das Innenrohr an dessen proximalem Ende mit einem die optischen Bauelemente hermetisch abschließenden Gehäuse fest und dichtend zu einer zweiten Baueinheit zusammengefügt ist.

Aufgrund der Tatsache, daß die äußeren Bauelemente, nämlich Außenrohr, Optikkopf und die Beobachtungselemente (Okularmuschel, oder Adapter, oder integrierte Miniaturkamera) zu einer festen Baueinheit zusammengefügt sind, können mechanische Einwirkungen, sei es durch mechanische Stöße beim Ablegen oder Handhaben, sei es durch Dehnvorgänge bei den Wärmeschocks, zu keinen Relativverschiebungen der Bauelemente in diesem steifen Zusammenbau führen. Dieser ist auf Dauer formstabil und die einzelnen Bauelemente Außenrohr, Optikkopf und Okularmuschel bleiben fest und unverrückbar aneinandergefügt. Aufgrund der Tatsache, daß die Zusammenfügung derart bewerkstelligt wird, daß diese Teile dicht untereinander zusammengefügt sind, ist eine Baueinheit geschaffen, bei der von der Außenseite her, mit Ausnahme der beiden endseitigen Öffnungen keine Feuchtigkeit, Gase oder sonstige Kontaminationen eintreten können.

Aufgrund der Tatsache, daß das Innenrohr an seinem proximalen Ende mit einem die optischen Bauelemente hermetisch abschließenden Gehäuse fest und dichtend zu einer zweiten Baueinheit zusammengefügt ist, sind die optischen Elemente von der Außenwelt hermetisch abgeschlossen, so daß keine Kontaminationen, seien es gasförmiger oder flüssiger Art, in das optische System eintreten können.

Diese beiden Baueinheiten sind am distalen Ende starr und dicht miteinander verbunden. Am proximalen Ende ist dann das Innenrohr bzw. das proximale Ende des hermetisch abschließenden Gehäuses im Optikkopf abgestützt. Die bei den Temperaturschocks erfolgenden Längenausdehnungen bzw. Längenschrumpfungen der beiden langerstreckten Baueinheiten können nun, ausgehend von dem fixen distalen Verknüpfungspunkt dieser beiden Baueinheiten untereinander, ungestört nebeneinander stattfinden. Ungleiche Längenausdehnungen der Baueinheiten können nun durch Relativbewegungen zwischen diesen ermöglicht werden. Diese Relativbewegung führt einerseits nicht zu einer Beeinträchtigung der mechanischen Stabilität des Endoskops, denn diese wird ja wesentlich durch den äußeren umhüllenden Zusammenbau der ersten Baueinheit aus Außenrohr, Optikkopf und Okularmuschel sichergestellt. Diese Relativbewegung kann auch nicht zu Undichtigkeiten im optischen System führen, da die innere zweite Baueinheit in sich hermetisch abgeschlossen ist. Zwischen der Außenseite der inneren zweiten Baueinheit und der Innenseite der äußeren ersten Baueinheit werden, wie an sich bekannt, im Optikkopf Abdichtmaßnahmen beispielsweise über O-Ringe getroffen, um beim Autoklavieren kein Wasser oder Dampf eindringen zu lassen. Sollte dies aber dennoch einmal erfolgen, ist das weder der mechanischen Stabilität abträglich, noch hat es einen negativen Einfluß auf das optische System, da ja dieses als solche hermetisch abgeschlossen.

Somit wird die Aufgabe vollkommen gelöst.

In einer Ausgestaltung der Erfindung ist der proximale Endbereich der zweiten Baueinheit schwimmend im Optikkopf abgestützt.

Diese Maßnahme hat den Vorteil, daß diese schwimmende Lagerung, die dennoch dicht gegenüber Eintritt von Autoklavierdampf oder Flüssigkeit ist, eine verklemmfreie Längenausdehnung bei den Temperaturschocks erlaubt und auch ermöglicht, radial einwirkende mechanische Schocks oder Stöße beim Ablegen oder versehentlichen Herabfallen eines Endoskops derart aufzunehmen oder zu verteilen, daß keine Beeinträchtigung des optischen Systems erfolgt. Das optische System beinhaltet zahlreiche Linsen, beispielsweise relativ lange Stablinsen aus Glasmaterialien, die bei intensiven mechanischen Schocks möglicherweise brechen könnten. Die schwimmende Lagerung erlaubt solche Schocks sanfter oder gedämpfter aufzunehmen, so daß dadurch die Lebensdauer des Linsensytems erheblich erhöht ist.

In einer weiteren Ausgestaltung der Erfindung ist der proximale Endbereich der zweiten Baueinheit ortsfest am Optikkopf abgestützt und ist mit Dehnungsmerkmalen versehen.

Im Gegensatz zu der voran beschriebenen Ausführung, bei der sich die innere zweite Baueinheit nach proximal verschieben kann, ist diese Möglichkeit hier durch den Anschlag nicht gegeben, der Längenausgleich wird durch die Dehnungsmerkmale erfüllt. Hier kann das proximale Ende der inneren Baueinheit dauernd an einer ganz bestimmten Stelle gehalten werden, dieses Ende ist üblicherweise mit einem Glasfenster oder Linsen verschlossen, um den Durchblick durch das Innenrohr zu gewährleisten. Nachjustieren der Optik wegen Relativbewegungen sind nicht mehr notwendig. Die notwendige Längenausdehnung wird über die Dehnungsmerkmale bewerkstelligt.

In einer besonders bevorzugten Ausgestaltung dieser Bauweise besteht das Dehnungsmerkmal in einer faltenbalgartigen Ausgestaltung der Wand des Gehäuses.

Bei den Dehnungs- oder Schrumpfvorgängen nimmt der Faltenbalg die notwendigen Formveränderungen auf, so daß die anderen Bauteile, insbesondere die Linsen, in einer unveränderbaren Relativstellung zueinander verbleiben.

In einer weiteren Ausgestaltung der Erfindung erfolgt die feste und dichte Verbindung sowohl der einzelnen Bauelemente der beiden Baueinheiten untereinander als auch die Verbindung der Baueinheiten am distalen Ende miteinander durch Verlöten, Verschweißen oder Verkleben.

Diese Maßnahme hat den Vorteil, daß durch gängige Arbeitsverfahren nicht nur der entsprechend mechanische Verbund zwischen den zu verbindenden Teilen geschaffen wird, sondern auch die entsprechend dichte Verbindung, die sowohl mechanischen Schocks als auch den Temperaturschocks auf Dauer widerstehen.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend an Hand einiger ausgewählter Ausführungsbeispiele in Zusammenhang mit den beiliegenden Zeichnungen näher beschrieben und erläutert. Es zeigen:
- Fig. 1: einen Längsschnitt der beiden Baueinheiten eines erfindungsgemäßen Endoskops in getrenntem, noch nicht miteinander verbundenen Zustand, wobei der Übersicht halber die optischen Bauelemente weggelassen sind,
- Fig. 2: den Zusammenbau der beiden Baueinheiten von Fig. 1, wobei ebenfalls der Übersicht halber die optischen Bauelemente wie Linsen weggelassen sind, und
- Fig. 3: eine der Schnittdarstellung von Fig. 2 vergleichbare Schnittdarstellung eines weiteren Ausführungsbeispiels samt den optischen Bauelementen.

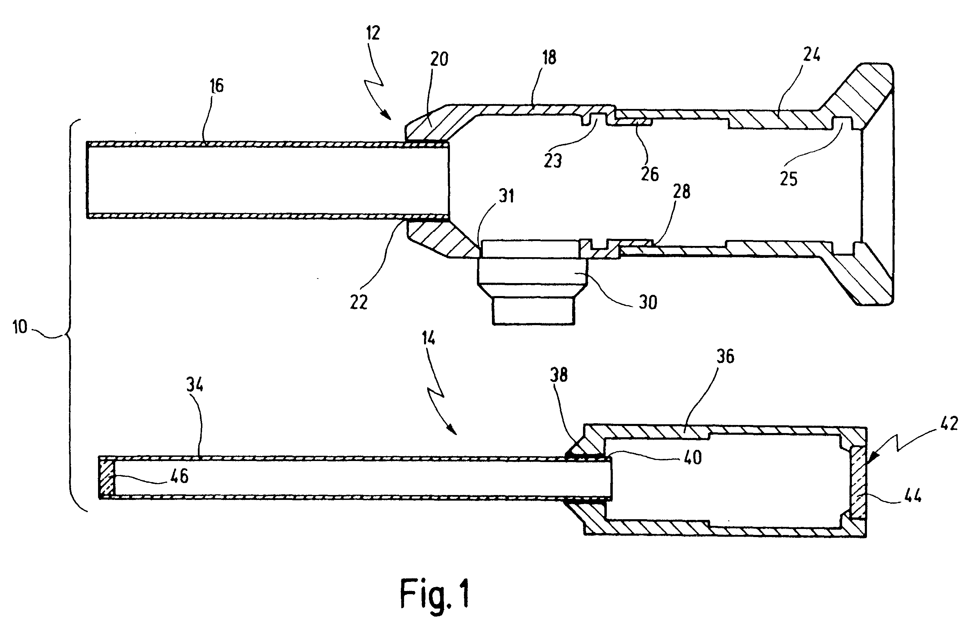

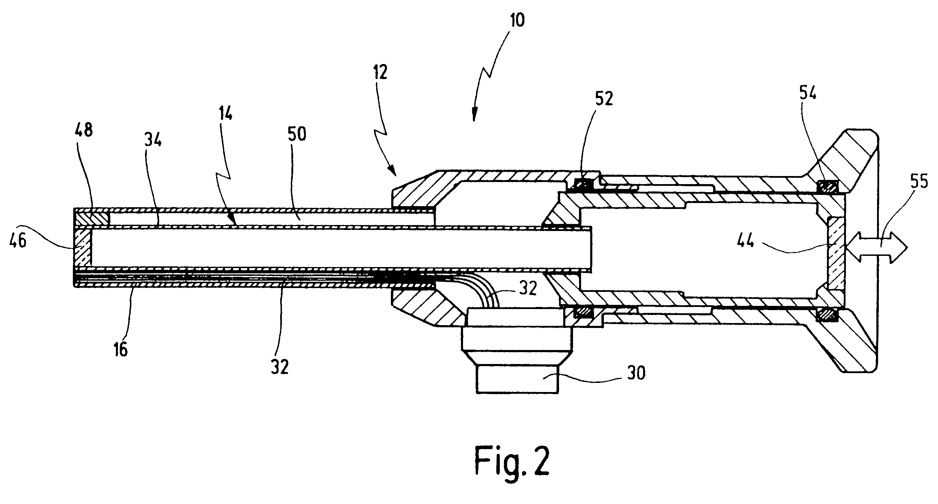

Ein in den Figuren 1 und 2 gezeigtes Endoskop ist in seiner Gesamtheit mit der Bezugsziffer 10 versehen.

Das Endoskop 10 besteht im wesentlichen aus einer ersten Baueinheit 12 und einer zweiten Baueinheit 14, wie sie in Fig. 1 getrennt untereinander liegend dargestellt sind.

Die erste Baueinheit 12 weist ein Außenrohr 16 auf, dessen Länge je nach Einsatzzweck des Endoskops variiert, in der Darstellung als relativ kurzes Außenrohr 16 dargestellt ist.

Das Außenrohr 16 ist am proximalen Ende mit einem Optikkopf 18 verbunden.

Der Optikkopf 18 weist ein etwa hohlzylindrisches Gehäuse auf, an dessen proximalen Ende ein Montageflansch 20 vorgesehen ist.

Das proximale Ende des Außenrohrs 16 ist in den Flansch 20 eingeschoben und durch Verlöten ist eine mechanisch stabile und absolut gas- und wasserdichte Verbindung 22 zwischen diesen Bauteilen geschaffen.

Der Optikkopf 18 ist am proximalen Ende mit einer Okularmuschel 24 verbunden.

Dazu steht proximal ein Rohrflansch 26 vor, auf den die Okularmuschel 24 aufgeschoben ist. Die Verbindung 28 in diesem Bereich erfolgt über Verschrauben und Verkleben.

Am Optikkopf 18 ist ein radial vorspringender Stutzen 30 angebracht, der ebenfalls über eine gas- und flüssigkeitsdichte Verbindung 31 verklebt ist.

An der Innenseite des Optikkopfes 18 ist im Bereich des Rohrflansches 26 eine Ringnut 23 ausgebildet, eine entsprechende Ringnut 25 ist im Bereich des proximalen Endes der Okularmuschel ausgebildet.

Durch diese Ausgestaltung entsteht ein mechanisch sehr steifes und widerstandsfähiges Gebilde in Form der ersten Baueinheit 12.

Die zweite Baueinheit 14 besteht aus einem Innenrohr 34, das am proximalen Ende mit einem Gehäuse 36 versehen ist. Das Gehäuse 36 weist distal einen Flansch 38 auf, in den das proximale Ende des Innenrohrs 34 eingeschoben ist. Eine gas- und flüssigkeitsdichte Verbindung 40 entsteht durch Verlöten dieser beiden Elemente miteinander. Das Gehäuse 36 ist hohlzylindrisch. Am distalen Ende ist das Innenrohr 34 mit einer durchsichtigen Scheibe 46 verschlossen, die eingelötet ist. Das proximale Ende des Gehäuses 36 ist mit einer Scheibe 44 versehen, die ebenfalls gas- und flüssigkeitsdicht eingelötet ist, wie das auch bei der Scheibe 46 der Fall ist.

Die Scheiben 44 und 46 bilden endseitige Begrenzungen einer im Inneren des Gehäuses 36 bzw. des Innenrohrs 34 aufgenommenen Optik, die der Übersicht halber hier nicht weiter dargestellt ist. Durch den zuvor beschriebenen Zusammenbau der zweiten Baueinheit 14 entsteht ein hermetisch abgeschlossenes Gebilde, in dem die gesamte Optik, gegenüber Eindringen von Kontaminationen geschützt aufgenommen ist.

Bei der Montage wird die zweite Baueinheit 14 vom proximalen Ende her in die erste Baueinheit 12 eingeschoben, bis die distalen Enden von Außenrohr 16 und Innenrohr 34 etwa auf gleicher Höhe zu liegen kommen.

Wie aus Fig. 2 zu entnehmen, werden diese im distalen Bereich mechanisch fest und dichtend über eine Verbindung 48 miteinander verbunden. Die Verbindung 48 kann beispielsweise aus einem Ring bestehen, der mit der Außenseite des Innenrohrs 34 bzw. der Innenseite des Außenrohrs 16 verlötet wird. Zwischen Innenrohr 34 und Außenrohr 16 ist nunmehr ein Ringraum 50 geschaffen, in den beispielsweise Lichtfasern 32 über den Stutzen 30 bis an das distale Ende geführt werden. Die Verbindung 48 sieht dann entsprechende Lichtaustrittsöffnungen vor.

In die Ringnuten 23 bzw. 25 sind O-Ringdichtungen 52 bzw. 54 eingelegt, die einen dichten Abschluß zwischen Außenseite des Gehäuses 36 und der Innenseite von Optikkopf 18 bzw. Okularmuschel 24 schaffen.

Der Außendurchmesser des Gehäuses 36 ist dabei etwas geringer als der lichte Innendurchmesser von Optikkopf 18 bzw. Okularmuschel 24.

Dadurch ist eine schwimmende Abstützung bzw. Lagerung des Gehäuses 36 gewährleistet, die bei Temperaturänderungen eine Längenausdehnung ermöglicht, wie das in Fig. 2 durch den Doppelpfeil 55 angedeutet ist. Beim Dehnen schiebt somit das proximale Ende des Gehäuses 36 der inneren zweiten Baueinheit 14 sich proximal Richtung Ende der Okularmuschel 24.

Die O-Ringdichtungen 52 und 54 erlauben diese Bewegungen und nehmen auch radial einwirkende mechanische Stöße zum Teil auf.

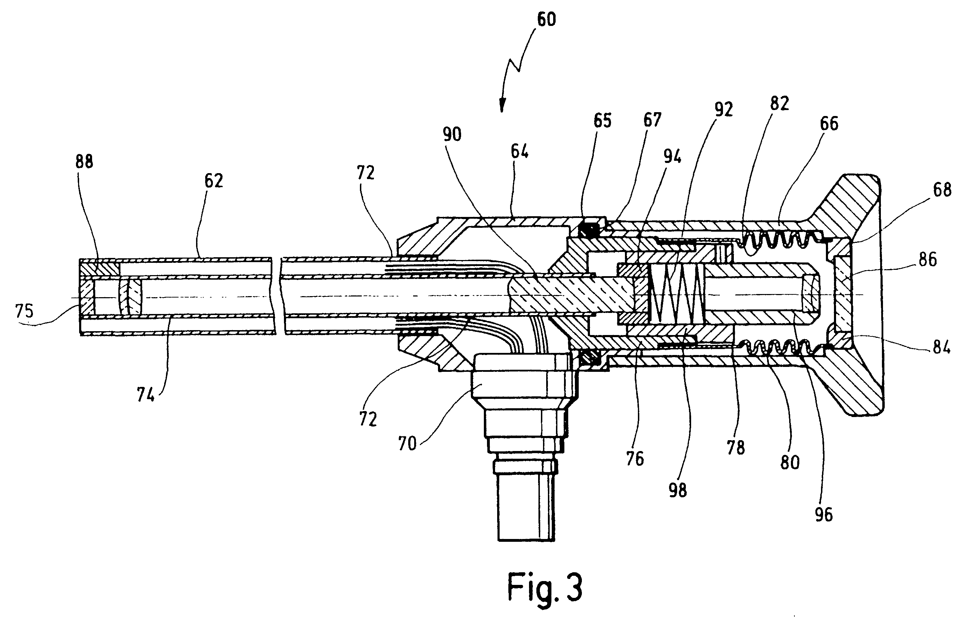

Bei einem weiteren in der Fig. 3 gezeigten Ausführungsbeispiel eines erfindungsgemäßen Endoskops 60 ist ebenfalls eine erste äußere Baueinheit aus einem Außenrohr 62, einem Optikkopf 64 und einer Okularmuschel 66 vorhanden, die ebenfalls, wie zuvor beschrieben, aneinandergefügt sind. Ein radial vorstehender Stutzen 70 dient gleichermaßen zur Zufuhr von Lichtfasern 72.

An der Stelle, an der die zuvor in Zusammenhang mit Fig. 1 beschriebene Ringnut 23 ausgebildet ist, ist hier eine entsprechende Ringnut 65 vorgesehen.

Am proximalen Ende der Okularmuschel 66 ist ein Anschlag 68 vorgesehen, dessen Sinn und Zweck später erläutert wird.

Die innere zweite Baueinheit setzt sich ebenfalls aus einem Innenrohr 74 und einem Gehäuse 76 zusammen. Distal ist das Innenrohr wieder über eine Scheibe 75 abgeschlossen.

Im Gegensatz zum in Zusammenhang mit Figuren 1 und 2 gezeigten Ausführungsbeispiel ist ein proximaler Abschnitt 78 des Gehäuses 76 als relativ dünne Wand 80 ausgebildet, die die Form eines Balgs 82 einnimmt. Das proximale Ende des faltenartig gewellten Balges 82 ist mit einem Ring 84 fest verlötet, der mittig eine Scheibe 86 trägt.

Der Ring 84 sitzt am Anschlag 68, somit ist das proximale Ende der inneren zweiten Baueinheit festgelegt.

Wie zuvor beschrieben, ist das distale Ende des Innenrohres 74 über eine Verbindung 88 mit dem distalen Ende des Außenrohres 62 verbunden.

Die erforderliche Längenausdehnung wird nunmehr durch die Verformung des Balges 82 aufgenommen.

Aus der Schnittdarstellung von Fig. 3 ist zu entnehmen, daß im Innenrohr 74 zahlreiche Stablinsen 90 aufgenommen werden, die über eine schraubenlinienförmige Feder 92 aneinandergedrückt gehalten werden. Dazu drückt die Feder 92 eine Kappe 94 auf den Zusammenbau der Stablinsen 90. Am gegenüberliegenden Ende stützt sich die Feder 92 auf einem Rohrfortsatz 96 ab, der fest mit einem Zwischengehäuse 98 verbunden ist, der die Feder 92 umgibt.

Diese an sich bekannte Anordnung erlaubt eine geringfügige Relativbewegung der Stablinsen 90 untereinander, so daß keine Abriebstellen entstehen. Der Druck der Feder 92 hält jedoch die Stablinsen 90 aneinandergelegt.

In der Ringnut 65 ist eine O-Ringdichtung 67 aufgenommen, so daß der Zusammenbau aus Innenrohr 74 und Gehäuse 76 in diesem Bereich etwa schwimmend abgestützt ist, nur am proximalen Ende fest am Anschlag 68 sitzt.

Diese Bauweise erlaubt Längendehnungen bzw. -kürzungen bei Temperaturschocks unter Beibehaltung der Relativlage der Linsen des optischen Systems.

Sowohl die O-Ringdichtung 67 als auch der faltenartige Balg 82 erlauben mechanische Stöße oder thermische Schocks soweit aufzunehmen und zu verteilen, ohne daß die Stablinsen 90 aus Glasmaterialien einer Bruchgefahr unterliegen.

Sollte dennoch eine Justierung oder eine Relativverstellbarkeit des Linsensystems, sei es zum Fokussieren oder zum Justieren, gewünscht sein, so kann dies über berührungslose Kupplungen, beispielsweise über Magnetkupplungen, erfolgen.

So kann beispielsweise im Bereich des Rohrfortsatzes 96 ein innerer Magnetring vorgesehen sein, der mit einem über die Außenseite der Okularmuschel 66 angebrachten äußeren Magnetring kraftschlüssig in Drehverbindung steht. In diesem Falle ist dann ein Gewinde vorgesehen, das eine Drehbewegung des inneren Magnetringes in eine Axialverschiebung des Rohrfortsatzes 96 umsetzt. Diese Verstellmöglichkeit kann ohne Eingriff in das Bauprinzip der beiden Baueinheiten verwirklicht werden.

## Patentansprüche

1. Endoskop, mit einem Außenrohr (16, 62), das mit einem Optikkopf (18, 64) verbunden ist, welcher eine Okularmuschel oder eine Adaptereinrichtung für ein Kamerasystem oder eine integrierte Miniaturkamera (24, 66) trägt, ferner mit einem im Außenrohr (16, 62) angeordneten und sich in den Optikkopf (18, 64) hinein erstreckenden und dort abgestützten Innenrohr (34, 74), das optische Bauelemente (42, 90) trägt, wobei Außenrohr (16, 62) und Innenrohr (34, 74) am distalen Ende des Endoskops starr und dichtend miteinander verbunden (48, 88) sind, **dadurch gekennzeichnet, daß** Außenrohr (16, 62), Optikkopf (18, 64) und Okularmuschel (24, 66) oder die Adaptereinrichtung für eine Kamera oder die integrierte Miniaturkamera zu einer festen und zwischen diesen Teilen dichtenden, nach außen jedoch nicht abgeschlossenen ersten Baueinheit (12) zusammengefügt sind, daß das Innenrohr (34, 72) an dessen proximalem Ende mit einem die optischen Bauelemente (42, 90) hermetisch abschließenden Gehäuse (36, 76) starr und dichtend zu einer zweiten Baueinheit (14) zusammengefügt ist.

2. Endoskop nach Anspruch 1, **dadurch gekennzeichnet, daß** ein proximaler Endbereich der zweiten Baueinheit (14) schwimmend im Optikkopf (18, 64) abgestützt ist.

3. Endoskop nach Anspruch 1, **dadurch gekennzeichnet, daß** ein proximaler Endbereich der zweiten Baueinheit (14) ortsfest am Optikkopf (64) abgestützt ist, und mit Dehnungsmerkmalen versehen ist.

4. Endoskop nach Anspruch 3, **dadurch gekennzeichnet, daß** die Dehnungsmerkmale in einer faltenbalgartigen Ausgestaltung der Wand (80) des Gehäuses (76) bestehen.

5. Endoskop nach einem der Ansprüche 1 - 4, **dadurch gekennzeichnet, daß** die starre und dichte Verbindung sowohl der einzelnen Bauelemente der beiden Baueinheiten (12, 14) untereinander als auch die Verbindung der beiden Baueinheiten (12, 14) am distalen Ende miteinander durch Verlöten, Veschweißen oder Verkleben erfolgt ist.

## Claims

1. Endoscope, having an outer tube (16, 62) that is joined to an optical head (18, 64) that carries an eyepiece cup or an adapter device for a camera system or an integrated miniature camera (24, 66), also having an inner tube (34, 74), arranged in the outer tube (16, 62) and extending into the optical head (18, 64) and supported there, that carries optical components (42, 90), the outer tube (16, 62) and inner tube (34, 74) being rigidly and sealingly joined (48, 88) to one another at the distal end of the endoscope, **characterized in that**
the outer tube (16, 62) and the optical head (18, 64), and the eyepiece cup (24, 66) or the adapter device for a camera or the integrated miniature camera, are fitted together to form an immovable first module (12) that is sealed among these parts but not closed off from the outside; and at its proximal end the inner tube (34, 72) is immovably and sealingly fitted together with a housing (36, 76) that hermetically closes off the optical components (42, 90), to form a second module (14).

2. Endoscope of Claim 1, **characterized in that** a proximal end region of the second module (14) is supported in floating fashion in the optical head (18, 64).

3. Endoscope of Claim 1, **characterized in that** a proximal end region of the second module (14) is supported in stationary fashion on the optical head (64) and is equipped with expansion features.

4. Endoscope of Claim 1, **characterized in that** the expansion features consist in a bellows-like configuration of the wall (80) of the housing (76).

5. Endoscope of anyone of Claims 1 through 4, **characterized in that** both the immovable sealed join among the individual components of the two modules (12, 14) and the join between the two modules (12, 14) at the distal end are accomplished by soldering, welding, or adhesive bonding.

## Revendications

1. Endoscope, comportant un tube extérieur (16, 62), qui est assemblée à une tête optique (18, 64), qui porte une bonnette d'oculaire ou un dispositif d'adaptation pour un système de caméra ou une caméra miniature (24, 66) intégrée, comportant en outre un tube intérieur (34, 74), qui est agencé dans le tube extérieur (16, 62) et s'étend à l'intérieur de la tête optique (18, 64) et s'y appuie, et qui porte des composants optiques (42, 90), le tube extérieur (16, 62) et le tube intérieur (34, 74) étant assemblés (48, 88) l'un à l'autre de manière rigide et étanche au niveau de l'extrémité distale de l'endoscope, **caractérisé en ce que** le tube extérieur (16, 62), la tête optique (18, 64) et la bonnette d'oculaire (24, 66) ou le dispositif d'adaptation pour une caméra ou la caméra miniature intégrée sont assemblés pour former une première unité (12) fixe et assurant l'étanchéité entre ces parties, mais non fermée vers l'extérieur, **en ce que** le tube intérieur (34, 72) au niveau de son extrémité proximale, est assemblé de manière rigide et étanche à un boîtier (36, 76), fermant hermétiquement les composants optiques (42, 90), pour former une deuxième unité (14).

2. Endoscope selon la revendication 1, **caractérisé en ce qu'**une zone d'extrémité proximale de la deuxième unité (14) est en appui flottant dans la tête optique (18, 64).

3. Endoscope selon la revendication 1, **caractérisé en ce qu'**une zone d'extrémité proximale de la deuxième unité (14) est en appui localement fixe contre la tête optique (64) et possède des attributs d'allongement.

4. Endoscope selon la revendication 3, **caractérisé en ce que** les attributs d'allongement sont réalisés par la conception en forme de soufflet de la paroi (80) du boîtier (76).

5. Endoscope selon l'une quelconque des revendications 1 à 4, **caractérisé en ce que** l'assemblage rigide et étanche entre les différents composants des deux unités (12, 14), de même que l'assemblage des deux unités (12, 14) l'une à l'autre au niveau de l'extrémité distale sont réalisés par brasage, soudage ou collage.
